# EUROPEAN PATENT APPLICATION

(11) **EP 3 327 429 A1**
(43) Date of publication of application: **30.05.2018**
(21) Application number: 16200915.3
(22) Date of filing: 28.11.2016
(51) Int. Cl.: G01N 22/00, G01N 33/12

(54) **METHOD AND SYSTEM FOR DETERMINING MATERIALS OF A MATERIAL COMPOSITION**

(71) Applicant: Vestel Elektronik Sanayi ve Ticaret A.S., 45030 Manisa (TR)
(72) Inventor: ABBAK, Mehmet, 45030 Manisa (TR); ÖCAL, Ömür, 45030 Manisa (TR); MEMISOGLU, Görkem, 45030 Manisa (TR)
(74) Representative: Ascherl, Andreas

(57) **Abstract**

The present invention refers to a method for determining materials of a material composition (2). The inventive method comprises the steps: Providing a material composition (2); Biasing the material composition (2) with an electric field, wherein at least one parameter of the electric field is varied; Detecting values representing the dielectric property of at least one of the materials of the material composition (2) in dependency of the varied parameter; Transforming the detected values in representing values; Matching the representing values with data base values provided in a data base, wherein the data base values are representing specific materials; Outputting data or signals representing the material or materials matching with the data base values. The present invention further refers to a system or device for executing the before mentioned method.

## Description

The present invention refers to a method for determining materials of a material composition according to claim 1 and according to claim 14 to a system or device for executing the before mentioned method respectively to a system or device for determining materials of a material composition.

### Background of the Invention

Document EP 0121340 A2 discloses an optical method and apparatus for instantaneously determining the thickness of thin films which is well suited for instantaneously measuring the thickness of semiconductor layers deposited in a deposition apparatus. The thickness is calculated from the interference pattern of a spectrogram generated when continuous wavelength illumination is reflected from the thin film.

In a study published in 2015, ultimate frequencies where differentiation, amongst distinct kind of meats, is maximized between 0.5 GHz - 50 GHz is analyzed, and some potential biomarker frequencies to investigate has been determined to discriminate and resolve the species of different type of meat (Zainal Abidin, Z., Omar, F. N., Biak, D. R. A., & Man, Y. C. (2016). Alternative for rapid detection and screening of pork, chicken, and beef using dielectric properties in the frequency of 0.5 to 50 GHz. International Journal of Food Properties, 19(5), 1127-1138). In this work measurements are performed with standard measurement kits.

Food can be e.g. considered as a material composition, since many types of food are consisting of different materials respectively compositions.

Examination of foods with various ingredients and with different ratios can become very ambiguous. Getting the exact proportion of the additives is complicated in food industry. There are many products which are a mixture of different ingredients with various ratios in food industry, especially in the processed meats, like salami, sausage, soudjouk etc.. The main problem is ratios of the ingredients could not be detected easily. However, fast and accurate analysis of food is essential for healthy servings.

### Object of the Invention

It is thus the object of the present invention to provide a method and system or device for determining materials of a material composition in fast and reliable manner.

### Description of the Invention

The before mentioned object is solved by a method for determining materials of a material composition according to claim 1. The inventive method preferably comprises at least the steps: Providing a material composition; Biasing the material composition with an electric field, wherein at least one parameter of the electric field is varied; Detecting values representing an electric property, in particular the dielectric property, of at least one of the materials of the material composition in dependency of the varied parameter; Transforming the detected values in representing values; Matching the representing values with data base values provided in a data base, wherein the data base values are representing specific materials; Outputting data or signals representing the material or materials matching with the data base values.

Thus, in this invention, a dielectric property of the materials which is the energy handling capability under an influence of electric field source is considered. This method is therefore based on the measurement of scattering parameters related to the probes, respectively detection means which can give much faster results compared to chemical or biological analysis.

This solution is beneficial since the invention can be applied to various areas where homogeneity of the specific material is controversial, and therefore the dialectical properties of the material. In many cases, dielectric properties of the materials can show analytical sign related to the features of the substance, due to fact that, dielectric properties are directly related to the physical properties of the material in the atomic-scale.

This invention can be used to detect any prospective feature of the material composition which is in any relation with electrical properties. This invention allows the easy, fast and homogenous measurement of small samples that is under examination.

Dielectric constants of the materials present the electrical properties of that object under test, and it can be any property like amount of the moisture or bulk density of the material. The dielectric constant of a chicken breast is known as er = 58 at 1 GHz. A dielectric constant of a beefburger is know as between εr = 38 - 40 at 2.43 GHz which includes with different ratios of fat, bread, salt.

Furthermore, different parts of the meats have been measured at 27.12 MHz. Results are: Beef er = 70.5, Lamb (leg) εr = 77.9, Pork (shoulder) εr = 69.6, Chicken (breast) εr = 75, Turkey (breast) εr = 73.5, Pork (back) εr = 12.5. Homogeneous mixtures of these meats have electrical properties with same ratio.

Further preferred embodiments are described in the following parts of the description or are defined by dependent claims.

According to a preferred embodiment of the present invention the step of biasing the material composition with an electric field is carried out by means of a plurality of detection means, wherein the detection means are coaxial probes which are coupled with a two-port or n-port network analyzer.

For this reason, the potential applications of this invention can be expanded, to materials respectively material compositions wherein the examination of the material surface is suitable to be measured with a coaxial probe system. The coaxial probes can be referred to as detection means. One of the essential problems in terms of food is the non-equal heterogeneous mixture of the foods. For that reason, single probe result is not efficient and adequate, therefore multiple detection means, in particular probes, are provided. The coaxial probe system preferably comprises at least 5 or at least 10 or at least 15 or at least 20 or at least 25 coaxial probes.

The detection means are operated according to a further preferred embodiment of the present invention between 0,01 GHz and 100 GHz, in particular between 0,5 GHz and 50 GHz.

Thus, the usage of multi detection means, which are preferably configured as microwave probes, in particular as coaxial microwave probes, is preferred.

Therefore, according to the present invention usage of one-port to N-port scattering matrix measurement is enabled. Different algorithms can be applied according to the number of the ports. The method or system can work healthy with 1-port measurement. However, by increasing the number of the ports, accuracy of the evaluation regarding to the object under test increases. Also, increasing the number of the ports reduces time to evaluate the object under test, by gathering the information from different parts of the sample at the same time.

By using one-port measurement dielectric properties of the touched material with coaxial probe is obtained by calibrating the probe previously. In the calibration, preferably three different measurements should be done as a reference. These are e.g. short-circuit, open circuit, and load measurements. As a load a known material, in terms of electrical properties, is measured. A reference load represents a material with known electrical properties. Water, in particular distilled water or demineralized water, is a good candidate to be used as a reference load.

In multi-port measurements, calibrations are preferably made between the all ports using e.g. TRL (through-reflect-line) and/or SOLT (short-open-load-thru). Depending on the contrast between the calibration measurements and the measurement of the object, dielectric constant of the material is calculated.

After accurately measuring the dielectric properties results are classified, preferably by using different machine learning techniques or data base comparisons.

According to a further preferred embodiment of the present invention the detected values represent complex data representing magnitude data and phase data. Additionally, or alternatively complex impedance of multiple, in particular each, ports is obtained. Preferably are the reflection coefficients of multiple, in particular each, detection means processed by a first algorithm for determining a dielectric constant value for each detection means. Additionally, or alternatively transmission data between multiple, in particular each, ports is retrieved, wherein the transmission of emitted electromagnetic waves at multiple, in particular each, detection means and/or the alteration of the electromagnetic waves in terms of phase and/or magnitude is processed by a second algorithm for determining the dielectric constant value for each detection means. This embodiment is beneficial since due to the different algorithms very precise features can be obtained. The algorithms can be applied separately or combined. It is further conceivable that only one of the algorithms is processed.

The detection means are according to a further preferred embodiment homogenously, in particular in the same distance to each other, or heterogeneously applied to the material composition. This solution is beneficial since a homogenous application on the material composition allows a precise prediction of the material property of the material composition between the detection means. The distance between two detection means is preferably smaller than 15cm or 10cm or 5cm, in particular smaller than 3cm or 2cm or 1 cm or the distance between two detection means is preferably larger than 1cm or 2cm or 3cm or 4cm or 5cm.

The detection means are according to a further preferred embodiment of the present invention calibrated for each measurement and/or in a specific time interval, in particular with a TRL or SOLT type calibration. This embodiment is beneficial since due to a calibration step the overall results can be enhanced.

The herein described method preferably comprises an image processing step. The images of that image processing step are preferably captured by a camera and processed, in particular by the computing unit, as image data. The image data herby preferably represents at least sections of the material composition. Preferably at least one of boarder, size, location and/or shape of the material composition is/are detected in that image processing step. The image data is hereby preferably analyzed with an image analyzation algorithm.

A location for setting up a physical contact between at least one detection means and the material composition for performing the analysis is computed according to a further preferred embodiment of the present invention in dependency of the image processing. In particular multiple locations for setting up a physical contact between multiple detection means and the material composition are computed in dependency of the image processing.

According to a further preferred embodiment of the present invention a relative movement between the detection means respectively all or a majority or defined number of detection means at once and the material composition is carried out. The relative movement is preferably caused by an actuation of at least one actuator element, in particular a X-/Y-actuator or a rotating actuator, arranged for moving the detection means and/or a support. The actuator element is preferably operated in dependency of the image processing step, in particular timewise in dependency of the image processing step. The relative position between the support and the detection means is preferably changed in such a manner that a predefined or maximum number of detection means is in contact with the material composition. It is also possible that the positions of contact between the detection means and the material composition are changed by a relative movement between the material composition and the detection means. The detection means can hereby contact the material composition in a first step in a first section of the material composition and in a second step in a second section of the material composition. It is possible that the detection means are contacting the material composition in a plurality of sections and thus a plurality of steps for setting up the contact in the different sections can be carried out. A plurality of different sections can comprise two or more than two or three or up to three or more than three or five or up to five or more than five or ten or up to ten or more than ten or fifteen or up to fifteen or more than fifteen different sections. Each section preferably defines a specific surface area of the material composition. It is possible that individual sections are overlapping.

The values representing the dielectric properties of multiple materials of the material composition are determined for the contacting area between multiple, in particular each, detection means and the material composition and/or for the sections of the material composition between the contacting areas.

The material composition comprises or consists according to a further preferred embodiment of the present invention of a mixture of materials, in particular organic and/or inorganic materials and/or water and/or salt, in particular food, wherein the data base values are representing at least a predefined number of preferably organic and/or inorganic materials. Preferably are the majority (in volume %) of the materials organic materials and highly preferably are all materials organic materials.

Thus, this invention preferably refers to a food ingredients detector respectively a method or device or system for determining organic materials of a material composition, in particular food. Preferably, food comprising multiple sorts of meat, in particular form different animals, can be analysed.

Therefore, a major application of the herein described method and system or device is in the food industry, where ratio of the ingredients can be controversial. With this invention, ratios of the ingredients in the variety meat type of foods can be conveniently obtained. The multi probes of the system or device are distributed on the material composition, in particular food, to make measurements from the different regions of the material composition.

The present invention further refers to a system or device for executing the before mentioned method. Such a system or device for determining materials of a material composition preferably comprises at least a data base for providing values corresponding to the dielectric properties of at least multiple, in particular each, materials of the material composition, a computing unit for transforming detected values in representing values and for matching the representing values with data base values provided in a data base, an analyzing unit at least comprising multiple detection means preferably coupled with the computing unit, a tray or support for holding the material composition, preferably an actuator element for moving the detection means and/or the tray respectively the support, and preferably an image capturing unit for capturing images of the material composition and/or one or multiple detection means, wherein the detection means and/or the actuator element is/are preferably actuated respectively moved in dependency of an image processing of the images captured by the image capturing unit.

Since the herein described device or system is in particularly useful for detecting organic materials it can be considered as food ingredient sensor.

The detection means are according to a further preferred embodiment of the present invention coaxial probes, which are preferably coupled with a n-port network analyzer, which is preferably coupled with the computing unit.

Further benefits, goals and features of the present invention will be described by the following specification of the attached figures, in which exemplarily components of the invention are illustrated. Components of the systems, devices and methods according to the invention, which match at least essentially with respect to their function can be marked with the same reference sign, wherein such components do not have to be marked or described in all figures.

In the following the invention is just exemplarily described with respect to the attached figures.

### Brief Description of the Drawings

- Fig. 1a: shows a schematic and perspective view of the inventive device for determining materials of a material composition;
- Fig. 1b: shows a side view of the inventive device shown in Fig. 1a; and
- Fig. 2: shows a schematic illustration showing the functional connections between multiple units of the inventive system.

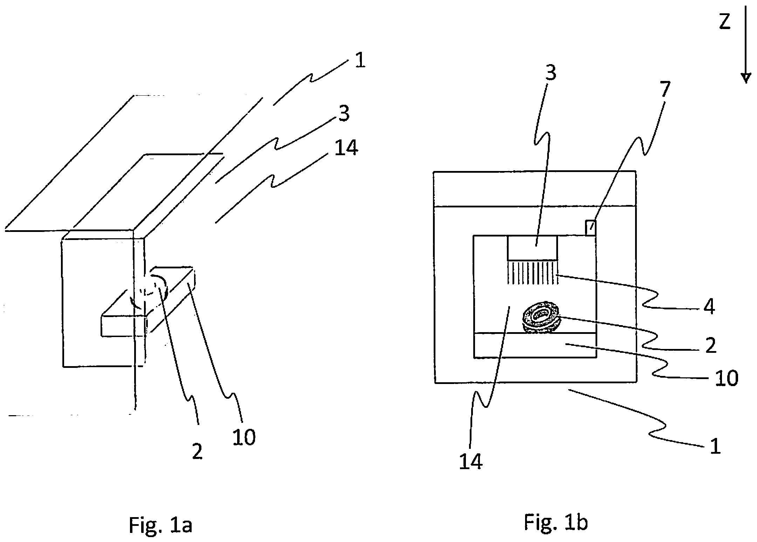

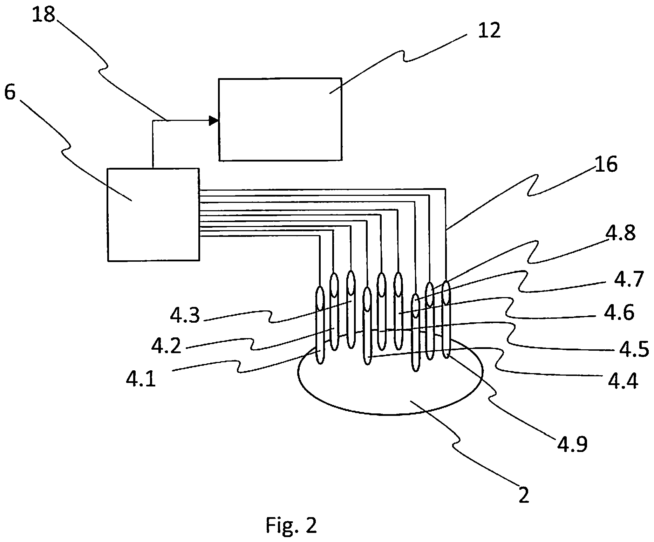

Fig. 1a and 1b show an apparatus, in particular a device 1, for executing the method according to the present invention. The device 1 preferably comprises a housing that defines a receiving space respectively operation space 14 for receiving a material composition which has to be analyzed. The device 1 further comprises an analyzing unit. That analyzing unit preferably comprises multiple detection means 4. Said detection means 4 are preferably coaxial probes that can be operated between 0,01 GHz and 100 GHz, in particular between 0,5 GHz and 50 GHz, and with an impedance of 20Ω to 70Ω, in particular with 50Ω. The detection means 4 are preferably arranged by means of a detection means support 3 above support 10 and are highly preferable movable with that detection means support 3 in Z-direction and/or in X-/Y-direction. Furthermore, a support 10 for holding the material composition 2 can be provided. Said support can be stationary or movable, in particular a X-/Y-table or a rotation table. It is further possible that an actuator element for moving the detection means 4 and/or the support 10 is provided. The analyzing unit preferably comprises an image capturing unit 7 for capturing images of the material composition 2 and/or one or multiple detection means 4. The image data represents at least sections of the material composition 2, wherein that information is preferably evaluated and/or utilized for the actuation or control of the actuation element 8. Thus, the actuator element 8 respectively motor can be actuated in dependency of image data captured by the image capturing unit 7 and/or in dependency of commands provided by a computing unit 12 (cf. fig. 2).

It should be noted that system is preferably enclosed to filter the uncontrollable outside effects and electromagnetic interference. Also, in the side view (Fig. 1b) the image capturing unit 7, in particular camera or CCD-camera or 3D-camera, which is preferably used for optimum distribution of the probes on the sample respectively the material composition is indicated above support 10 and preferably inside the operation space, in particular on the right top corner of device 1.

Said material composition 2 preferably comprises or consists of a mixture of organic materials, in particular food.

Fig. 2 shows a schematic illustration of the data gathering process that is executed according to the inventive method. Multiple detection means 4.1-4.9 are contacting the material composition 2, in particular the surface of the material composition 2. The material composition 2 is biased respectively subjected with an electric field by the detection means 4.1-4.9. At least one parameter of the electric field is preferably varied over time. The detecting means are configured for detecting multiple detecting values respectively detecting signals which are representing an electric property, in particular the dielectric property, of at least one and preferably of multiple or of all of the materials of the material composition 2. The detecting values or signals are transferred via connection 16 from the individual detection means 4 to a data collecting means 6, in particular a two-port or n-port network analyzer 6. It can be possible that the two-port or n-port network analyzer 6 is equipped with a control means (not shown) that transforms the detected values or signals into representing values. Alternatively, the collecting means 6 transfers the collected signals or data to a computing unit 12, in particular a PC, notebook, server, etc. and the detected values or signals are transformed by the computing unit 12 into representing values, in particular by applying specific algorithms. The computing unit 12 further performs a matching of the representing values with data base values provided in a data base. The data base can be part of the computing unit. The data base values are preferably representing specific materials, like different kinds of meat from different kinds of animals and/or just different kind of meat from the same kind of animal and/or different kinds of vegetables and/or fruits and/or bacteria and/or germs and/or other organic and/or inorganic materials, in particular by one or more electric or dielectric properties of the respective material.

Data or signals representing the material or materials matching with the data base values are preferably outputted and/or stored.

The connections 16 and 18 can be set up by cables or wireless.

A distribution of the detection means 4, in particular coaxial probes, is preferably obtained by placing them on a moving tray in two axis system.

In this invention, homogenous distribution of the multiple detection means 4.1-4.9 respectively probes are provided, according to the type and size of the food samples. The image capturing unit 7 preferably executes a camera control system, preferably by using standard machine vision techniques and by using image processing algorithms features like border, size and/or location of the material composition, in particular food or meat sample, is analysed. Beside uniform distribution any different types of distributions, like normal distribution, can be easily implemented in this system to obtain the most accurate results.

The collecting means 6, in particular network analyser, can preferably measure the scattering parameters of the connected N-port, and a N by N matrix of the complex data of the ports can be obtained. Complex data preferably contains phase and magnitude of the scattering parameters of the ports. This data is preferably used in two terms; first of all, complex impedance of each port is obtained. Secondly, transmission data between each port is retrieved. Two different types of algorithms, for two different types of data are preferably applied to obtain the dielectric properties of the food. In the first technique complex reflection coefficients at each probe is going to be used to calculate the dielectric constant. Secondly, transmission of the electromagnetic waves through each port, and their alteration in terms of phase and magnitude is going to be employed in determining dielectric constant. These analyses can be done on wide range of frequency or narrowband, according to the time period, sensitivity, and/or total cost requirements of the systems.

Obtained dielectric permittivity values regarding to the detection means 4 points respectively probe points and to the space between the detection means 4 respectively probes can be used by machine learning algorithm for classification, to simply the classify the measured food types.

Thus, as a measurement device, multiple detection means 4, in particular open ended coaxial probes can be used. The diameter of the detection means 4 respectively coaxial probes can be changed according to the application. Detection means 4 respectively probes are preferably matched to the input impedance of 50Ω, to be easily used with standard VNA's (Vector Network Analyser) which are commercially accessible. Commercial VNA's can be utilized to decrease the cost of the system, custom hardware and software solution can be designed to measure complex scattering parameters, including phase and magnitude of electrical networks. Also, to extend the capabilities of a two-port vector network analyzer cheaper software controlled n-port switches can be used. So, a custom two-port network analyzer can be enhanced to n-port vector network analyzer, with a dramatically reduced costs compared to commercial ones. One of the drawbacks of using switches is going to be increased measurement time. Also, to reduce the cost of the system number of the ports, so the detection means 4 respectively probes, can decreased, which is going to decrease the accuracy and sensitivity of the system. To minimize the errors, before each measurement or between a specific time interval, calibration of the network analyzer respectively collecting means 6 should be performed. TRL or SOLT type calibration techniques and commercial standard calibration kits are suitable for this purpose.

Therefore, the present invention refers to a method for determining materials of a material composition 2. The inventive method comprises the steps: Providing a material composition 2; Biasing the material composition 2 with an electric field, wherein at least one parameter of the electric field is varied; Detecting values representing the dielectric property of at least one of the materials of the material composition 2 in dependency of the varied parameter; Transforming the detected values in representing values; Matching the representing values with data base values provided in a data base, wherein the data base values are representing specific materials; Outputting data or signals representing the material or materials matching with the data base values. The present invention further refers to a system or device for executing the before mentioned method.

This invention provides a fast, accurate and straightforward examination of the ingredient of the foods. This is especially challenging problem in processed meat industry, where different ratios of distinct meats can be used, to exactly measure the proportions of the ingredients. In this invention multiple probes for detecting the electrical properties, which is directly related to the physical properties of the food, are uniformly distributed on the food under investigation.

It has to be understood that the herewith disclosed method and device can also be executed for determining material compositions different to food, e.g. like organic waste or soil.

### Reference numbers

- 1: Device or system for determining materials of a material composition
- 2: material composition
- 3: detection means support
- 4: detection means
- 6: two-port network analyzer or n-port network analyzer or collecting means
- 7: camera
- 10: support
- 12: computing unit
- 14: operation space
- 16: connections between network analyzer and detection means
- 18: connection between network analyzer and computing unit

## Claims

1. Method for determining materials of a material composition (2),
at least comprising the steps:
Providing a material composition (2);
Biasing the material composition (2) with an electric field,
wherein at least one parameter of the electric field is varied;
Detecting values representing an electric property, in particular the dielectric property, of at least one of the materials of the material composition (2) in dependency of the varied parameter;
Transforming the detected values in representing values;
Matching the representing values with data base values provided in a data base,
wherein the data base values are representing specific materials;
Outputting data or signals representing the material or materials matching with the data base values.

2. Method according to claim 1,
**characterized in that**
the step of biasing the material composition (2) with an electric field is carried out by means of a plurality of detection means (4), wherein the detection means (4) are coaxial probes which are coupled with a two-port or n-port network analyzer (6).

3. Method according to claim 1 or 2,
**characterized in that**
the detection means (4) are operated between 0,01 GHz and 100 GHz, in particular between 0,5 GHz and 50 GHz.

4. Method according to claim 1, 2 or 3,
**characterized in that**
the detected values represent complex data representing magnitude data and phase data.

5. Method according to claim 4,
**characterized in that**
complex impedance of multiple, in particular each, ports is obtained,
wherein the reflection coefficients of multiple, in particular each, detection means (4) are processed by a first algorithm for determining a dielectric constant value for each detection means (4).

6. Method according to claim 4 or claim 5,
**characterized in that**
transmission data between multiple, in particular each, ports is retrieved,
wherein the transmission of emitted electromagnetic waves at multiple, in particular each, detection means (4)
and/or
the alteration of the electromagnetic waves in terms of phase and/or magnitude
is processed by a second algorithm for determining the dielectric constant value for multiple, in particular each, detection means (4).

7. Method according to any of the previous claims,
**characterized in that**
the detection means (4) are homogenously, in particular in the same distance to each other, applied to the material composition (2).

8. Method according to any of the previous claims 2 to 7,
**characterized in that**
the detection means (4) are calibrated for each measurement or in a specific time interval, in particular with a TRL or SOLT type calibration.

9. Method according to any of the previous claims,
**characterized by**
an image processing step,
wherein the images are captured by a camera (7) and processed as image data,
wherein the image data represents at least sections of the material composition (2),
wherein at least one of boarder, size, location and/or shape of the material composition (2) is/are detected in an analyzing step,
wherein the image data is analyzed with an image analyzation algorithm in that image processing step.

10. Method according to claim 9,
**characterized in that**
a location for setting up a physical contact between at least one detection means (4) and the material composition (2) for performing the analysis is computed, in particular multiple locations for setting up a physical contact between multiple detection means (4) and the material composition are computed, in dependency of the image processing.

11. Method according to claim 9 or 10,
**characterized by**
a relative movement between the detection means (4) and the material composition (2),
wherein the relative movement is caused by an actuation of at least one actuator element arranged for moving the detection means (4) and/or a support (10) for holding the material composition (2),
wherein the actuator element is operated in dependency of the image processing step.

12. Method according to any of the previous claims,
**characterized in that**
detecting values representing the dielectric properties of multiple materials of the material composition (2) are determined for the contacting area between multiple, in particular each, detection means (4) and the material composition (2) and/or for the sections of the material composition between the contacting areas.

13. Method according to any of the previous claims,
**characterized in that**
the material composition (2) comprises or consists of a mixture of organic materials, in particular food, wherein the data base values are representing at least a predefined number of organic materials.

14. System or device (1) for determining materials of a material composition (2),
at least comprising
a data base for providing values corresponding to the dielectric properties of at least multiple, in particular each, materials of the material composition (2),
a computing unit (12) for transforming detected values in representing values and for matching the representing values with data base values provided in a data base,
an analyzing unit at least comprising
multiple detection means (4) coupled with the computing unit (12)
a support (10) for holding the material composition (2),
an actuator element for moving the detection means (4) and/or the support (10),
and
an image capturing unit for capturing images of the material composition (2) and/or one or multiple detection means (4),
wherein the detection means (4) and/or the actuator element is/are actuated in dependency of an image processing of the images captured by the image capturing unit (7).

15. System or device (1) according to claim 14,
**characterized in that**
the detection means (4) are coaxial probes, which are coupled with a two or n-port network analyzer (6), which is coupled with the computing unit (12).
